# EUROPEAN PATENT APPLICATION

(11) **EP 2 878 261 A1**
(43) Date of publication of application: **03.06.2015**
(21) Application number: 14382480.3
(22) Date of filing: 27.11.2014
(51) Int. Cl.: A61B 5/00, A61B 5/11

(54) **A system for monitoring the state of a subject**

(30) Priority: 27.11.2013 ES 201331727
(71) Applicant: Centro Tecnologico del Mueble y La Madera de la Region de Murcia, 30510 Murcia (ES)
(72) Inventor: Maestre Ferriz, Rafael, 30510 Murcia (ES); Bleda Tomas, Andres Lorenzo, 30510 Murcia (ES); Santa Carvajal, Guadalupe, 30510 Murcia (ES); Pellicer Fernandez, Soledad, 30510 Murcia (ES); Garcia Ortega, Sergio, 30510 Murcia (ES)
(74) Representative: Isern-Jara, Nuria

(57) **Abstract**

A system for monitoring the state of a subject, specifically designed for installation in homes or rooms etc., comprising items of furniture (1) with at least one built-in sensor capable of measuring a physical parameter of the subject; a processing unit (3) linked to a piece of software for receiving an electrical signal from at least said sensor and a data communication module (4) for wirelessly transmitting the parameters obtained by at least said sensor. As such, this processing unit (3) generates a signal when it detects an anomalous value or pattern of behaviour linked to at least one parameter received by at least one sensor.

## Description

### OBJECT OF THE INVENTION

The present invention patent application seeks to register a system for monitoring the state of a subject, comprising notable innovations and advantages.

More specifically, the invention proposes the development of a system for monitoring the state of a subject, especially designed for installation in a home or room etc., which facilitates the control of the subject by means of the sensors distributed in items of furniture, such as chairs, sofas and beds, etc.

### BACKGROUND OF THE INVENTION

In modern day society, mainly due to an aging population and an increased number of people suffering from chronic conditions, there is a growing number of "dependent" people who need direct attention. Family members usually work and have other responsibilities that require all of the attention these people need. Moreover, the average family is unable to take on the financial costs needed to provide this assistance by way of specialised carers, owing to the expenses this entails.

Chairs especially adapted for use in office jobs, which make it possible to obtain information about the user's actions and needs, for example, are currently known about in the chair market. These chairs have built-in pressure sensors distributed at the surface of the seat and backrest area, thus making it possible to capture real time data about the chair and its occupant, in turn producing a pressure distribution map as a grey scale image. Furthermore, pattern recognition techniques, usually employed in computing and robotic vision, are used as one of the main analysis components applied to resolve posture problems acquired upon sitting. As such, the aim of this chair is to correct posture, the same not being used to assist users dependent on care within their own homes.

Diagnostic chairs used to control blood pressure are also known about. These chairs use EMFi sensors to obtain a ballistocardiograph and to broadcast information via radio waves from the chair to a computer. Although these chairs pertaining to the state of the art provide very valuable information at medical level, they are not designed to obtain contextual information that may prove useful in checking the behaviour of someone in their own home and checking that they are going about their daily routine as usual.

Furthermore, the applicant does not currently know of any system with all of the characteristics described in the present specification.

### DESCRIPTION OF THE INVENTION

The present invention was developed with the aim of providing a system for monitoring the state of a subject, configured as a novelty within the field of application, which resolves the limitations set out above, furthermore contributing other additional advantages that shall become clear as of the description provided below.

As such, the aim of the present invention is to provide a system for monitoring the state of a subject, specifically designed for installation in a home or room, etc., characterised by the fact that it comprises:
- at least one item of furniture including at least one built-in sensor, capable of measuring a physical parameter of the subject;
- a processing unit linked to a piece of software, in order to receive an electrical signal from at least said sensor and;
- a data communication module, for wirelessly transmitting the parameters obtained by at least said sensor;
wherein the processing unit generates an alarm signal when it detects an anomalous value linked to the parameter received by at least said sensor.

The parameter that generates the signal may constitute the measurement value obtained from the at least one sensor or from a sequence or combination of measurement values from at least said sensor.

According to another aspect of the invention, the signal generated is at least an alarm signal and/or at least an actuation signal, which acts on at least one item of furniture or control element.

Owing to these characteristics, it is possible to improve the quality of people requiring daily control, as a result of a chronic illness or physical limitations brought about by old age, for example, thereby reducing the need for human intervention, whilst maintaining continuous, non-invasive supervision, which does not make the user feel like they are being watched at any time, since the sensors are built into the furniture itself.

Another significant advantage is that the user does not have to fit any kind of device to themselves in order to ensure adequate control of at least one of their physical parameters and control is carried out in such a way that the user is unaware of it, since the user must simply sit on a chair, arm chair or sofa or lie down in bed, for example.

In a particularly preferred embodiment, the system includes a plurality of items of furniture, each one of which is provided with at least one built in sensor.

The item of furniture may consist of a bed, sofa or armchair, etc., a table provided with at least one drawer or a combination of the same.

The following types of detector may be used in the system, upon being built into the items of furniture set out above, either individually or in combination with one another:
- an accelerometer,
- a pressure sensor,
- a weight sensor,
- a temperature sensor capable of measuring the subject's temperature upon them coming into contact with the item of furniture,
- a humidity sensor,
- an opening/closure sensor (for example, magnetic means that act by way of a closed circuit when the magnetic means are coupled),
- a movement sensor,
- a sensor for detecting the amount of light and
- height and/or angle detection sensors.

As such, sensors capable of measuring weight may be built into an item of furniture in which the user may sit or lie down. For example, a load cell with a metal structure containing a strain gauge responsible for measuring weight may be used. These sensors may be used to detect the user's presence and/or movement.

Using accelerometers makes it possible to detect movement in the piece of furniture, as well as the intensity and frequency thereof. This information indicates the user's level of activity or rest, as well as indicating any kind of blow that may indicate the subject having potentially fallen and indicating problems regarding the intensity thereof. The accelerometer may also give information on the direction of the item of furniture and whether or not the same is no longer vertical or whether it has rotated or moved, all of which may indicate falls or blows according to the detection profile.

Using pressure sensors makes it possible to detect whether or not the item of furniture is occupied, in addition to measuring the subject's activity. As such, they serve a similar purpose to accelerometers, although they cannot detect loss of vertical position or blows in areas where no pressure sensors are located. It is also possible to capture information on posture when sitting or lying down, upon sensors being distributed on the surface with which the subject comes into contact.

The presence of temperature sensors makes it possible to obtain the user's temperature, in such a way that it is possible to monitor the user's temperature and detect anomalous temperature patterns, thereby generating an alarm to alert of the existence of an anomaly in the subject's parameters. It must be noted that these temperature sensors are located at points in the item of furniture that come into contact with the user.

In a preferred embodiment, the humidity sensor comprises at least two textile current conductor threads arranged in a piece of impermeable, breathable material in the area in which the subject is resting in a sitting or lying position. These humidity sensors serve to detect any spillage of liquid on the surface of the item of furniture, such that should the user suffer any kind of urinary incontinence, the same would be detected immediately. These sensors may be suitably built into the upholstery of the armchair or into the upholstery of the mattress, in the event of being used in a bed.

In a preferred embodiment, the height detection sensors consist of two infrared distance sensors, used to measure the height of the area in which the subject is resting their head in relation to the height of the area in which their feet are supported, in such a way that it is possible to establish parameters that describe possible risk of a fall as a result of the height or angle of the mattress base.

Another preferred embodiment for detecting the parameters set out above involves combining the use of a height sensor and angle sensor.

The light sensors found in the system serve to measure the amount of light there is in a certain room. These light measurement sensors work upon generating an output current that depends on incident visible light hitting the surface of the sensor. As a result, it is suitable for determining at what time of day a support light must be turned on for the user, when they get up from bed during the night and there isn't enough light for them to carry out their desired activity.

In the case of articulated beds, the use of height and/or angle sensors proves useful, since it is possible to control the movement of the bed and monitor the position thereof, in such a way that it is possible to prevent users who must spend a long time lying down in bed from developing sores. These sensors may be of the variety commercially available on the market, for example accelerometers with three axes capable of providing information of the angle at which one or more moving parts of an articulated bed are titled; gyroscopes; spin potentiometers and even infrared distance sensors.

Using presence sensors makes it possible to determine the subject's location in a certain item of furniture. The simplest presence or occupation sensors work as a kind of multi-way switch that closes upon the item of furniture being occupied, the user's very weight applying pressure to two electrical conductor sheets that creates contact and generates a signal indicating that the user's presence has been detected.

Opening/closure sensors are used as safety elements in the item of furniture or as indicators for doors and drawers that may be opened/closed by the user themselves, in order to determine a certain action having been carried out, for example taking medication. It is also possible to incorporate these sensors in safety barriers, including the side barriers of a bed, which prevent the user from falling out of bed. Opening or closing certain safety elements makes it possible to send information confirming that the user is trying to get up or has opened something voluntarily. The sensors selected for a specific embodiment described herein are of the magnetic variety that are usually open, such that when a magnetized object comes closer, this sensor closes and acts as a closed circuit.

Movement sensors are used to detect the user's presence in the room in which this sensor is located, thus making it possible to capture information on that person's habits, in addition to unusual changes that may indicate problems. In the specific embodiment described herein, the sensor used is of the passive infrared (PIR) variety and the item of furniture into which this sensor may be built may be a conventional bedside night table, not limiting the integration thereof into any other item of furniture. This sensor must be fitted in such a way that the angle of vision thereof spans the greatest area of the room possible, avoiding obstacles in this angle of vision, such as objects that may alter the response thereof.

In a preferred embodiment, the same is built into the front lower portion of the night table, thus meaning a housing must be made in the night table structure, in which the sensor is fitted and the associated wiring is directed to the same.

Other characteristics and advantages of the monitoring system object of the present invention shall be made clear as of the description of a preferred, non-exclusive embodiment, set out by way of non-limiting example, in the drawings below, wherein:

### BRIEF DESCRIPTION OF THE DRAWINGS

Figure 1.- Is a schematic diagram of the monitoring system according to the present invention,
Figure 2.- Is a schematic perspective view of an armchair that forms part of the system object of the invention,
Figure 3.- Is a schematic perspective view of a bed that forms part of the system object of the invention and
Figure 4.- Is a schematic view of a mattress for a bed, provided with humidity sensors.

### DESCRIPTION OF A PREFERRED EMBODIMENT

In view of the Figures mentioned above and in accordance with the numbering adopted therein, it is possible to observe a preferred embodiment of the invention, which comprises the parts and elements indicated and described in detail below.

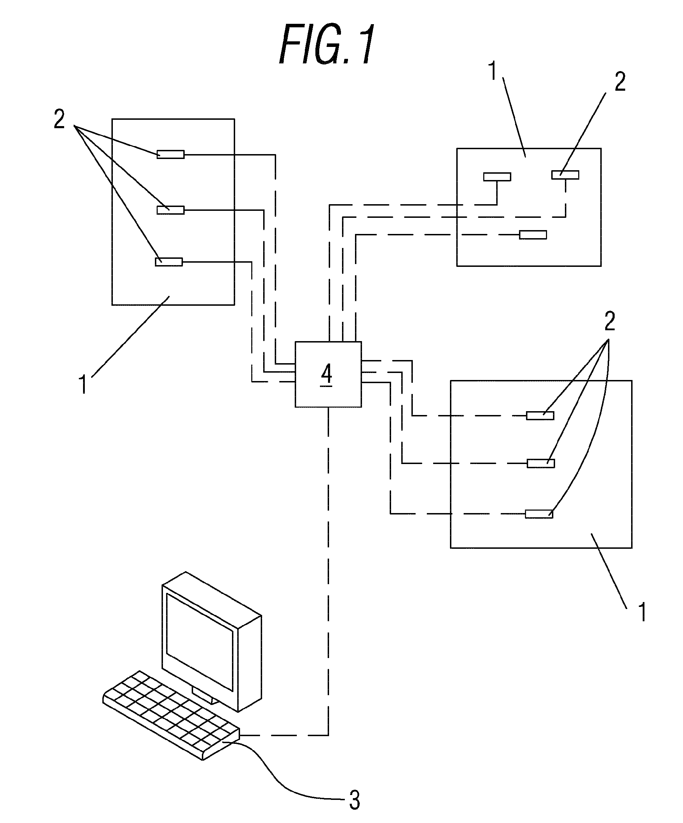

Indeed, as shown schematically in Figure 1, the monitoring system designed to give real time information on the state of a subject, specifically designed for installation in a home or room, etc., essentially comprises a plurality of items of furniture (1), a series of sensors being built into each one of the same, generally designated with the reference number (2), capable of measuring a physical parameter of the subject to be tracked (described in more detail later on); a data processing unit (3) linked to a piece of software for receiving an electrical signal from said sensors, which may be located at a remote point or within the home itself and a data communication module (4) for wirelessly transmitting the parameters obtained by said sensors. As such, the processing unit (3) is able to generate an alarm signal, signalling that the subject requires help from a third party (carer) because they are experiencing some kind of problem, when it detects an anomalous value linked to the parameter received by one of the sensors (2).

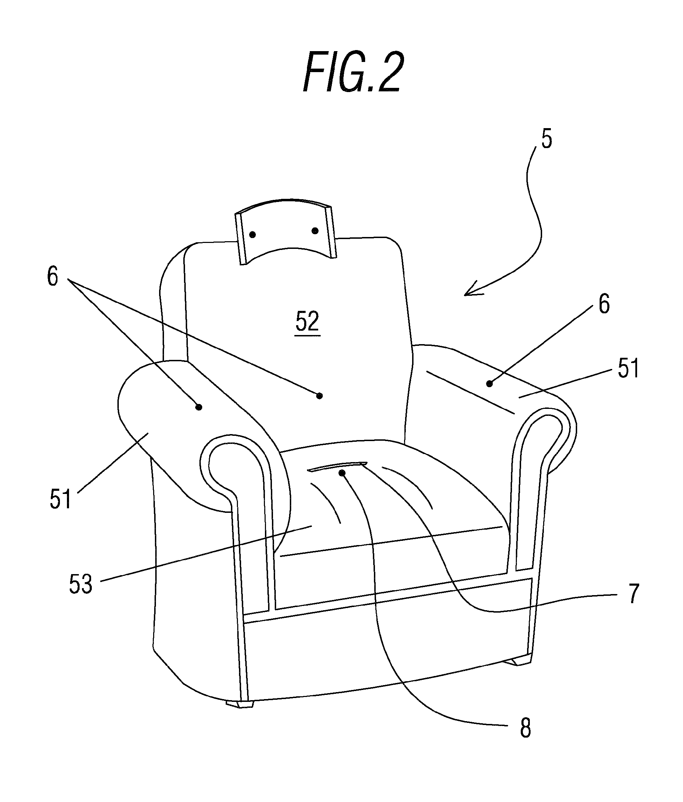

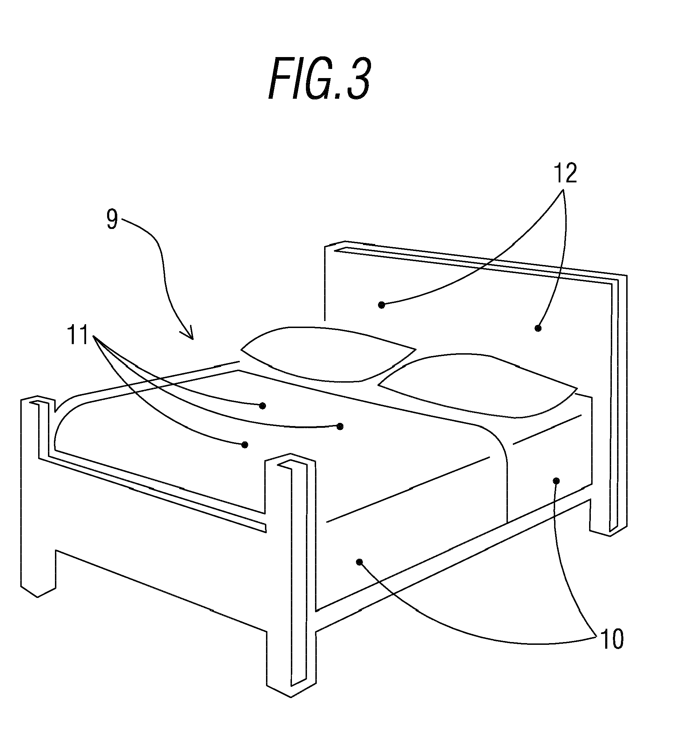

The items of furniture involved essentially consist of beds, sofas and armchairs etc., as well as tables, often known as bedside tables, with drawers, two specific embodiments corresponding to an armchair and a bed having been respectively represented in Figures 2 and 3, described below.

The following sensors (2) may be used in the system, which may be distributed between the various items of furniture in the home most often used by an elderly person:
- accelerometers,
- pressure sensors,
- weight sensors,
- temperature sensors for measuring the subject's temperature upon them coming into contact with the item of furniture,
- humidity sensors,
- opening/closure sensors, which in a preferred embodiment, may be magnetic means that act by way of a closed circuit when the magnetic means are coupled,
- movement sensors,
- sensors that detect the amount of light and
- height and/or angle detection sensors.

Now, with particular reference to an armchair, generally indicated with the reference number (5) in Figure 2, the same has weight sensors (not shown), which comprise a number of load cells built in between the legs of the chair (5) and the structure of the same, in such a way that all of the weight falls on the weight sensors. The user's weight will be the difference between the chair (5) when occupied and the chair when empty. In a specific embodiment, load cells have been built into the lower portion of the seat structure, such that each sensor is fastened by means of four holding points in a support beneath the sensor and, in the upper portion of the sensor, pressure is exerted with a surface such that the weight falls completely on the indicated point of the load cell.

Furthermore, as shown in Figure 2, three temperature sensors (6) are provided for, two of them being located on the arm rest (51) and the other being located in the back support area (52). These temperature sensors (6) are located underneath the upholstery, in such a way that they cannot be seen by the user, whilst nevertheless coming into contact with them through the very upholstery of the armchair (5) itself.

A humidity sensor (7) is also provided for, built into the seat area and more particularly located in an open housing (8) located in the seat area (53). Owing to the fact that the upholstery of the armchair is impermeable and to the slight gradient of the seat area, if a liquid were to be spilt, this liquid would run towards the open housing and come into contact with said sensor.

Now, with reference to the example of a bed shown in Figure 3, generally indicated by the reference number (9), load cells that form part of a number of weight sensors (10) are built in underneath the resting area of the main structure of the mattress base, such that the user's weight plus the weight of the mattress base and the mattress are supported by the weight sensors (10). The system may be calibrated without the user, in order to know how much it weighs alone and the difference in weight produced when the user occupies the same, thereby giving their true weight. Another possible embodiment, which is simpler than the latter, consists of building the weight sensors into the area between the legs and the bed.

A number of temperature sensors (11) have also been arranged in the bed (9), which are built into the mattress (not shown), more specifically being arranged underneath the upholstery covering the mattress in a number of pre-determined areas.

The possibility of making infrared temperature sensors (12) available, which measure the user's temperature without direct contact between the sensor and the user being necessary, must also be noted. Just like the infrared temperature sensor, this sensor monitors the user's temperature in the event of the system detecting anomalous temperature patterns, it being possible for the same to generate a specific alarm where necessary.

These infrared temperature sensors (12) may also be used to detect presence in the item of furniture (i.e. a sofa or bed, etc.,) since the temperature thereof would be higher when occupied by the user.

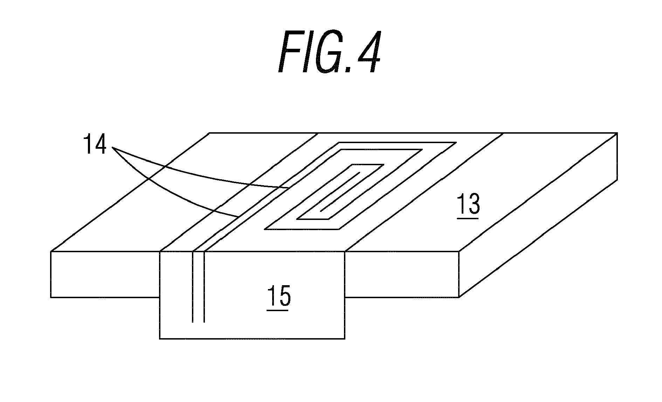

In Figure 4, it is possible to observe a specific embodiment of a humidity sensor in the mattress (13) of a bed, essentially comprising two textile current conductor threads (14) arranged in a piece of impermeable, breathable material (15) in the area in which the subject rests when sitting and/or lying down.

In addition, in an alternative embodiment of a bed structure, it is possible to incorporate a number of folding safety barriers (not shown), which prevent the user who is resting thereon from falling from the same involuntarily. Such barriers are linked to magnetic sensors, wherein the sensor serves as a multi-way switch and is fastened in the non-mobile area of the bed, to which the safety barriers are anchored to close the same. Moreover, the ferromagnetic elements are fastened to the four ends of the foldable safety barriers, very close to the closure ends, in such a way that they do not make operating the closure mechanisms more difficult or bumpy, whilst being capable of acting on the sensor object located in the fixed portion of the bed. As such, in the event of the user being detected to open the safety barriers at night time, a warning signal would be sent with the aim of a third party (for example a carer) coming to assist the user, in order to check their situation.

Greater detail was not entered into as regards the sensors used for various items of furniture described herein, since sensors currently available in the market may be mounted.

The details, shapes, dimensions and other complementary elements, as well as the materials used to produce the monitoring system of the invention, may be substituted for other, technically equivalent alternatives where convenient, provided that they do not diverge from the essence of the invention or the scope defined by the claims included below.

## Claims

1. A system for monitoring the state of a subject, especially designed for installation in homes or rooms etc., **characterised by** the fact that it comprises:
- at least one item of furniture (1) including at least one built-in sensor capable of measuring a physical parameter of the subject,
- a processing unit (3) linked to a piece of software for receiving an electrical signal from at least said sensor and
- a data communication module (4) for wirelessly transmitting the parameters obtained by at least said sensor;
wherein the processing unit (3) generates a signal when it detects an anomalous value or pattern of behaviour, linked to at least one parameter received by at least one sensor.

2. The monitoring system according to claim 1, **characterised by** the fact that the parameter that generates the signal consists of the measurement value from at least said sensor.

3. The monitoring system according to claim 1, **characterised by** the fact that the parameter that generates the signal consists of a sequence or combination of measurement values from at least said sensor.

4. The monitoring system according to claim 1, **characterised by** the fact that the signal generated is an alarm signal.

5. The monitoring system according to claim 1, **characterised by** the fact that the signal generated is at least an actuation signal, which acts on at least one item of furniture or control element.

6. The monitoring system according to claim 1, **characterised by** the fact that it includes a plurality of items of furniture (1), each one of which is provided with at least one built-in sensor.

7. The monitoring system according to claim 1, **characterised by** the fact that the item of furniture consists of a bed, sofa, armchair or similar.

8. The monitoring system according to claim 1, **characterised by** the fact that the sensor consists of an accelerometer.

9. The monitoring system according to claim 1, **characterised by** the fact that the sensor consists of a pressure sensor.

10. The monitoring system according to claim 1, **characterised by** the fact that the sensor consists of a weight sensor.

11. The monitoring system according to claim 1, **characterised by** the fact that the sensor consists of a temperature sensor, capable of measuring the subject's temperature upon them coming into contact with the item of furniture.

12. The monitoring system according to claim 1, **characterised by** the fact that the sensor consists of a humidity sensor.

13. The monitoring system according to claim 1, **characterised by** the fact that the sensor consists of an opening/closure sensor, which acts on at least one portion of an item of furniture.

14. The monitoring system according to claim 1, **characterised by** the fact that the sensor consists of a movement sensor.

15. The monitoring system according to claim 1, **characterised by** the fact that the sensor consists of height and or/angle detection sensors.
